(19) Europäisches Patentamt
European Patent Office
Office européen des brevets

(11) Veröffentlichungsnummer : **0 105 024**
**B1**

(12) # EUROPÄISCHE PATENTSCHRIFT

(45) Veröffentlichungstag der Patentschrift : **30.10.85**

(51) Int. Cl.[4] : **C 07 D209/76**, C 08 F 12/32

(21) Anmeldenummer : **83810345.5**

(22) Anmeldetag : **02.08.83**

(54) **Substituierte ungesättigte bicyclische Imide und deren Polymeren.**

(30) Priorität : **05.08.82 CH 4715/82**

(43) Veröffentlichungstag der Anmeldung : **04.04.84 Patentblatt 84/14**

(45) Bekanntmachung des Hinweises auf die Patenterteilung : **30.10.85 Patentblatt 85/44**

(84) Benannte Vertragsstaaten : **CH DE FR GB IT LI NL**

(56) Entgegenhaltungen :
**US-A- 3 450 711**
**US-A- 3 879 349**
**US-A- 4 380 643**

(73) Patentinhaber : **CIBA-GEIGY AG**
**Postfach**
**CH-4002 Basel (CH)**

(72) Erfinder : **Renner, Alfred, Dr.**
**Im Marcoup 2**
**D-3280 Muntelier (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 105 024 B1

Jouve, 18, rue St-Denis, 75001 Paris, France

## Beschreibung

Die Erfindung betrifft mit Allyl oder Methallyl substituierte Bicyclo-[2.2.1]hept-5-en-2,3-dicarbonsäureimide, deren Herstellung und die daraus durch Erwärmen erhältlichen Polymeren.

Maleinimide und Bismaleinimide sowie N-Allyl-monomaleinimide sind bekannt.

Im US Patent 3 334 075 wird die Härtung von halogenierten olefinischen Gummipolymeren mit ausgewählten Polymaleinimidverbindungen, wie N,N'-m-Phenylen-bismaleinimid, beschrieben. Diese Polymaleinimide enthalten keine Allyl- oder Norbornenylgruppen.

Aus dem britischen Patent 1 277 790 sind harzbildende Zusammensetzungen bekannt, welche Maleinimid- oder Bismaleinimidderivate, wie N-Phenyl-maleinimid und Methylen-bis-(N-phenyl-maleinimid), enthalten. Keine dieser Verbindungen enthält Norbornenyl- oder Allylgruppen.

Im US Patent 3 839 358 wird ein Verfahren zur Herstellung von Bis-maleinimiden durch Umsetzung einer Bismaleinamidsäure mit dem Anhydrid einer niederen Carbonsäure in Gegenwart eines tertiären Amins, eines organischen Lösungsmittels und eines Nickelkatalysators beschrieben. Aus dem US Patent 4 229 351 ist ein Verfahren zur Herstellung von am Stickstoffatom aliphatisch substituierten Mono- und Bismaleinimiden bekannt. Weder im einen noch im anderen Patent wird die Herstellung von Verbindungen mit allylsubstituierten Norbornenylgruppen beschrieben oder nahegelegt.

Das US Patent 3 450 711 betrifft Bisimidverbindungen, hergestellt durch Umsetzung von Endo, cis-bicyclo[2.2.1]hept-5-en-2,3-dicarbonsäureanhydrid (= 5-Norbornen-2,3-dicarbonsäureanhydrid) mit ausgewählten organischen Diaminen. Diese Bisimide enthalten keine Allylsubstituenten und unterscheiden sich von den vorliegenden Verbindungen sowohl durch ihre Struktur als auch durch ihre chemische Reaktivität. Die Verbindungen gemäss diesem US Patent werden als Zwischenprodukte bei der Herstellung von Epoxidverbindungen verwendet.

Ferner ist bekannt, dass durch Addition von 3,3',4,4'-Benzophenon-tetracarbonsäuredianhydrid und Diaminodiphenylmethan in Gegenwart verschiedener zur Vernetzung und zur Endgruppenverkappung befähigter Verbindungen, wie gegebenenfalls chloriertes 5-Norbornencarbonsäureanhydrid und 5-Vinylphthalsäureanhydrid, Polyimid-Oligomere hergestellt werden können, die als Klebstoffe Anwendung finden [vgl. z. B. Polym. Eng. Sci., *22,* 9-14 (1982)]. Diese Polyimid-Oligomeren enthalten keine Allylgruppen.

Im US Patent 4 271 074 werden Silane, hergestellt aus Imidzwischenprodukten, z. B. auch N-Allyl-2,3-dimethyl-maleinimid, beschrieben. Die erfindungsgemässen Monomeren weisen eine Norbornenylgruppe, welche mit einer Allylgruppe substituiert ist, auf, sind daher strukturell ganz verschieden und durch dieses Patent nicht nahegelegt.

Die Herstellung der Ausgangsprodukte für die erfindungsgemässen Verbindungen wird im US Patent 3 105 839 beschrieben.

Die erfindungsgemässen allyl- oder methallylsubstituierten Bicyclo-[2.2.1]hept-5-en-2,3-dicarbonsäureimide sind wertvolle Ausgangsprodukte für Polymere mit ausgezeichneten Eigenschaften. Sie sind durch die folgende Formel I gekennzeichnet:

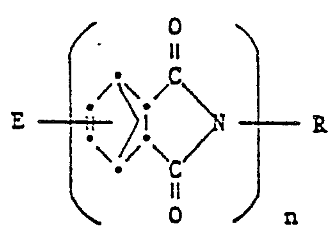

(I)

worin E Allyl oder Methallyl und n 1 oder 2 bedeuten und R, falls n 1 bedeutet, für Wasserstoff, Alkyl mit 1-12 C-Atomen, Alkenyl mit 3-6 C-Atomen, Cycloalkyl mit 5-8 C-Atomen, Aryl mit 6-10 C-Atomen oder Benzyl oder, falls n 2 bedeutet, für $-C_mH_{2m}-$ mit m = 2-20, Arylen mit 6-10 C-Atomen oder für eine Gruppe der Formel II

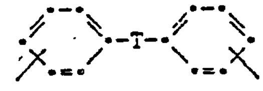

(II)

worin T Methylen, Isopropyliden, CO, O, S oder $SO_2$ bedeutet, steht.

Vorzugsweise bedeutet E die Allylgruppe.

R kann eine gerad- oder verzweigtkettige Alkylgruppe mit 1-12 C-Atomen bedeuten, wie Methyl, Aethyl, Isopropyl, n-Butyl, Isopentyl, n-Hexyl, 2-Aethyl-hexyl, n-Decyl und n-Dodecyl, vorzugsweise Alkyl mit 1-8 C-Atomen.

R kann auch eine geradkettige oder verzweigtkettige Alkenylgruppe mit 3-6 C-Atomen bedeuten, wie Allyl, Methallyl, 2-Butenyl und 3-Hexenyl, vorzugsweise Allyl.

Wenn R eine Cycloalkylgruppe bedeutet, kann es sich um eine Cyclopentyl-, Cyclohexyl-, Cyclohepty-l- oder Cyclooctylgruppe handeln, vorzugsweise um Cyclohexyl.

R in der Bedeutung einer Arylgruppe kann unsubstituiertes Phenyl oder eine durch eine oder zwei Methylgruppen substituierte Phenylgruppe sein, wie Tolyl oder Xylyl, oder auch Naphthyl. Bevorzugt ist die Phenylgruppe. Stellt R eine Gruppe $-C_mH_{2m}-$ dar, so kann es sich um geradkettige oder verzweigte Reste handeln, wie Aethylen, Propylen, Trimethylen, Tetramethylen, Hexamethylen, Octamethylen und Dodecamethylen. Bedeutet R eine Gruppe der Formel II, so ist diese vorzugsweise in 4,4'-Stellung an die N-Atome gebunden.

Vorzugsweise bedeutet R eine Gruppe $-(CH_2)_m-$ mit m = 2 bis 12.

R kann in der Bedeutung einer Arylengruppe mit 6-10 C-Atomen z. B. eine m-Phenylen-, p-Phenylen-, 1,3-Naphthylen-, 1,4-Naphthylen- oder 1,5-Naphthylengruppe bedeuten.

Wenn R eine Gruppe der Formel II bedeutet, steht T vorzugsweise für die Methylengruppe, O oder $SO_2$.

Bevorzugte Verbindungen der Formel I sind solche, worin E Allyl bedeutet und R, falls n = 1 ist, für Wasserstoff, Alkyl mit 1-8 C-Atomen, Allyl, Cyclohexyl, Phenyl oder Benzyl steht, oder, falls n = 2 ist, für $-(CH_2)_m-$ mit m = 2,12, m- oder p-Phenylen oder für eine Gruppe der Formel II steht, worin T die Methylengruppe, O oder $SO_2$ bedeutet.

Besonders bevorzugt sind Verbindungen der Formel I, worin E die Allylgruppe, n die Zahl 2 und R $-(CH_2)_2-$, $-(CH_2)_6-$

oder 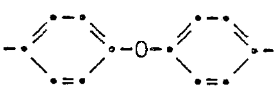 und insbesondere 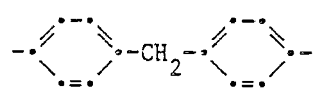

bedeuten.

Die erfindungsgemässen Imide können auf an sich bekannte Weise z. B. durch Umsetzen eines Anhydrids der Formel III

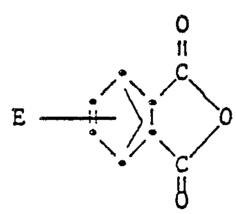 (III)

mit einer Verbindung der Formel IV

$$(H_2N)_n-R \qquad (IV),$$

worin E, R und n die unter Formel I angegebene Bedeutung haben, bei erhöhter Temperatur und unter Abdestillieren des bei der Reaktion entstehenden Wassers hergestellt werden. Sofern es sich bei den Verbindungen der Formel IV um Ammoniak oder niedrigsiedende Monoamine handelt, ist ein Ueberschuss dieser Reaktanden zu empfehlen. Diamine sind vorteilhaft in stöchiometrischem Verhältnis einzusetzen. Die Umsetzung kann ohne Lösungsmittel oder in Gegenwart eines inerten Lösungsmittels, das für die azeotrope Entfernung des Wassers verwendbar ist (Schleppmittel) erfolgen. Die Temperatur der Umsetzung kann zwischen 100 und 250 °C liegen. Bevorzugt werden die Imide der Formel I in der Schmelze bei einem Druck von höchstens 4 500 Pa bei Temperaturen zwischen 130 und 220 °C, insbesondere 180 und 220 °C, hergestellt.

Wie schon erwähnt, können die Ausgangsstoffe der Formel III gemäss dem in der US Patentschrift 3 105 839 beschriebenen Verfahren hergestellt werden, indem man Natrium-Cyclopentadienid mit einem Allyl- oder Methallylhalogenid umsetzt, worauf sich eine Diels-Alder-Reaktion mit Maleinsäureanhydrid anschliesst. Obgleich in der US Patentschrift angegeben wird, dass die Allylgruppe in 7-Stellung des bicyclischen Systems gebunden ist, zeigen neuere Untersuchungen, dass eine isomere Mischung in Bezug auf die Stellung der Allylgruppe und auch auf die Endo- und Exokonfiguration des Anhydridteils gebildet wird. Die isomeren Komponenten konnten mit üblichen Trennverfahren bisher nicht isoliert werden.

Die verwendeten Monoamine oder Diamine der Formel IV sind bekannt oder können nach an sich bekannten Verfahren hergestellt werden.

Die erfindungsgemässen Verbindungen sind flüssige oder niedrigschmelzende feste Stoffe, die zu festen Produkten mit hoher Glasumwandlungs-temperatur und Wärme- und Wasserbeständigkeit polymerisiert werden können. Diese Produkte können vielseitig angewendet werden, z. B. als Giessharze

oder Klebstoffe und insbesondere zur Herstellung von glasfaser- oder kohlenstoffaserverstärkten Kunststoffen, sowie als elektrische Isolierstoffe.

Die erfindungsgemässen Verbindungen können direkt verwendet und polymerisiert werden, oder sie können zunächst in einem organischen Lösungsmittel, wie Toluol, Xylol, Methyläthylketon, Aethylenglykol- monoalkyl- und -dialkyläthern mit 1-4 C-Atomen in den Alkylgruppen oder einem ähnlichen, in der Lackindustrie üblichen Lösungsmittel, gelöst werden. Solche Lösungen können als Imprägniermittel oder Beschichtungsmittel oder auch als Versandobjekt an den Verbraucher dienen.

Gegenstand der Erfindung sind auch die neuen Polymeren, die dadurch erhältlich sind, dass man ein Imid der Formel I während 6 bis 24 Stunden auf eine Temperatur zwischen 180 und 300 °C, vorzugsweise zwischen 200 und 250 °C, erhitzt. Dabei gilt in Bezug auf bevorzugte Bedeutungen von E, R und n das oben Angegebene. Besonders bevorzugt sind Polymere, die dadurch erhältlich sind, dass man ein Imid der Formel I, worin E die Allylgruppe, n die Zahl 2 und R die Gruppe

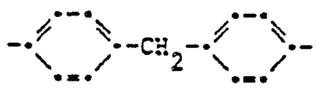

bedeuten, während 6-12 Stunden auf 240 bis 250 °C erhitzt. Katalysatoren, welche die Polymerisation wesentlich beschleunigen, wurden bisher nicht gefunden.

Selbstverständlich können den Imiden der Formel I inerte und stabile Stoffe, wie Füllmittel, Pigmente, Farbstoffe und andere Additive, zugegeben werden, bevor sie zu vernetzten Gebilden polymerisiert werden.

Beispiel 1 : Herstellung von Allyl-bicyclo[2.2.1]hept-5-en-2,3-dicarbonsäureimid.

Man erhitzt eine Mischung aus 102 g Allyl-bicyclo[2.2.1]hept-5-en-2,3-dicarbonsäureanhydrid, hergestellt gemäss Beispiel 1 der US Patentschrift 3 105 839, und 68,1 g 25 %iger wässriger Ammoniaklösung unter Rühren und Rückflusskühlung während 70 Minuten auf 95-102 °C. Danach destilliert man bei vermindertem Druck (6 133 Pa) Wasser und überschüssiges Ammoniak ab. Nach Erreichen einer Innentemperatur von 120 °C hat man 54 ml Ammoniakwasser abdestilliert. Es hinterbleibt ein orangefarbener, viskoser Rückstand, den man bei einem Druck von 13,3 Pa destilliert. Zwischen 132 und 135 °C gehen 62,07 g Allyl-bicyclo-[2.2.1]hept-5-en-2,3-dicarbonsäureimid über, was einer Ausbeute von 60 % der Theorie entspricht. Das Imid ist ein gelbes Oel mit einer Brechungszahl $n_D^{20}$ von 1,5 421 und einer Viskosität $\eta_{25}$ von 345 Pa.s bei 25 °C.

Analyse

| | | | |
|---|---|---|---|
| berechnet für $C_{12}H_{13}NO_2$ : | % C 70,9 | % H 6,45 | % N 6,90 |
| gefunden : | % C 70,7 | % H 6,6 | % N 6,9. |

IR-Spektrum : 1 620 $cm^{-1}$ cyclische Doppelbindung
1 640 $cm^{-1}$ Allylgruppe
1 725 $cm^{-1}$ Carbonylgruppe
1 772 $cm^{-1}$ Carbonyl im cyclischen Imid
3 390 $cm^{-1}$ NH-Schwingung.

Nach der Polymerisation während 24 Stunden bei 240 °C wird ein Festkörper mit einer Glasumwandlungstemperatur von 218,5 °C erhalten. Er zeigt die für die Doppelbindung charakteristischen IR-Absorptionsfrequenzen bei 1 620 und 1 640 $cm^{-1}$ nicht mehr.

Beispiel 2 : Allyl-bicyclo [2.2.1] hept-5-en-2,3-dicarbonsäure-N-methylimid.

Man erhitzt eine Mischung aus 204 g des in Beispiel 1 verwendeten Anhydrids und 113 g einer 33 gewichtsprozentigen Lösung von Methylamin in Alkohol unter Rühren und Rückflusskühlung auf 65 °C. Bei dieser Temperatur tritt eine exotherme Reaktion ein. Man kühlt die Mischung etwas, erwärmt während weiterer 2,5 Stunden auf 60-65 °C und destilliert den Alkohol, überschüssiges Methylamin und etwas Wasser ab (111 ml). Bei Erreichen einer Innentemperatur von 165 °C erniedrigt man den Druck sukzessive auf 6 665 Pa und hält die Mischung während 30 Minuten unter diesen Bedingungen.

Die Destillation des rotbraunen Rohprodukts ergibt bei 104-110 °C und 12 Pa 165,62 g (76 % der Theorie) eines gelben Oels mit $n_D^{20} = 1{,}526\,9$ ; $\eta_{25} = 1$ Pa.s.

Analyse

| | | | |
|---|---|---|---|
| berechnet für $C_{13}H_{15}NO_2$ : | % C 71,87 | % H 6,96 | % N 6,45 |
| gefunden : | % C 72 | % H 7,0 | % N 6,6. |

Die Polymerisation während 24 Stunden bei 240 °C ergibt einen Festkörper mit einer Glasumwandlungstemperatur (GUT) = 218,5 °C, der im IR-Spektrum keine C = C Absorptionsfrequenz bei 1 620 und 1 640 $cm^{-1}$ zeigt.

Beispiel 3 : Allyl-bicyclo[2.2.1]hept-5-en-2,3-dicarbonsäure-N-allylimid.

Man mischt 377,6 g Allyl-bicyclo[2.2.1]hept-5-en-2,3-dicarbonsäureanhydrid mit 131,9 g Allylamin

und erhitzt die Mischung am absteigenden Kühler, bis eine Innentemperatur von 175 °C erreicht ist. Man erhält 59 ml Destillat (Wasser und überschüssiges Allylamin). Bei der Destillation werden bei 40 Pa zwischen 120 und 123 °C 399,32 g (88,7 % der Theorie) eines hellgelben Oels mit folgenden Kenndaten erhalten : $n_D^{20}$ = 1,527 2 ; $\eta_{25}$ = 0,69 Pa.s.

Analyse

| | | | |
|---|---|---|---|
| berechnet für $C_{15}H_{17}NO_2$ : | % C 74,05 | % H 7,05 | % N 5,76 |
| gefunden : | % C 74,2 | % H 7,1 | % N 5,8. |

Die Polymerisation während 24 Stunden bei 240 °C ergibt einen Festkörper mit einer GUT > 290 °C. Im IR sind keine C = C-Absorptionsfrequenzen bei 1 620 und 1 640 $cm^{-1}$ feststellbar.

Beispiel 4 : Allyl-bicyclo[2.2.1]hept-5-en-2,3-dicarbonsäure-N-(2-äthyl-hexyl)-imid.

Man geht wie in Beispiel 3 beschrieben vor, wobei man aber 204 g Allyl-bicyclo[2.2.1]hept-5-en-2,3-dicarbonsäureanhydrid und 129 g 2-Aethyl-hexylamin verwendet. Während 4,5 Stunden werden bei 115 bis 160 °C und 4 000 Pa 17 g Wasser abgespalten. Man erhält 292,05 g (92,6 % der Theorie) einer Fraktion mit Kp = 160-164 °C bei 10,7 Pa ; $n_D^{20}$ = 1,5 038 ; $\eta_{25}$ = 0,68 Pa.s.

Analyse

| | | | |
|---|---|---|---|
| berechnet für $C_{20}H_{29}NO_2$ : | % C 76,15 | % H 9,27 | % N 4,44 |
| gefunden : | % C 76,2 | % H 9,4 | % N 4,4. |

Beispiel 5 : Allyl-bicyclo[2.2.1]hept-5-en-2,3-dicarbonsäure-N-cyclohexylimid.

102 g Allyl-bicyclo[2.2.1]hept-5-en-2,3-dicarbonsäureanhydrid und 100 g Cyclohexylamin werden bei 4 000 Pa bis auf 137 °C erhitzt. 59 ml Wasser und überschüssiges Cyclohexylamin werden abdestilliert. Die Destillation des Rückstandes bei 162-163 °C und 10,7 Pa ergibt 87,6 g (62 % der Theorie) eines Oels mit folgenden Eigenschaften : $n_D^{20}$ = 1,5 296 ; $\eta_{25}$ = 72,8 Pa.s.

Analyse

| | | | |
|---|---|---|---|
| berechnet für $C_{18}H_{23}NO_2$ : | % C 75,8 | % H 8,07 | % N 4,91 |
| gefunden : | % C 75,5 | % H 7,95 | % N 4,95. |

Beispiel 6 : Allyl-bicyclo[2.2.1]hept-5-en-2,3-dicarbonsäure-N-phenylimid.

In 102 g Allyl-bicyclo[2.2.1]hept-5-en-2,3-dicarbonsäureanhydrid tropft man unter Rühren 93 g Anilin ein. Hierbei steigt die Temperatur auf 80 °C. Man heizt bis auf 150 °C und reduziert den Druck auf 4 266 Pa. 9 ml Wasser und 46 ml Anilin destillieren ab. Die Destillation ergibt 82,37 g (60 % der Theorie) eines viskosen, gelben Oels mit Kp = 183 °C bei 12 Pa ; $n_D^{20}$ = 1,5 738 ; $\eta_{25}$ = 105,6 Pa.s.

Analyse

| | | | |
|---|---|---|---|
| berechnet für $C_{18}H_{17}NO_2$ : | % C 77,4 | % H 6,14 | % N 5,02 |
| gefunden : | % C 77,2 | % H 6,4 | % N 5,02. |

Die Polymerisation während 24 Stunden bei 240 °C ergibt einen Festkörper mit einer GUT von 230 °C. Im IR-Spektrum finden sich keine Absorptionsfrequenzen für

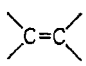 (1 620 und 1 640 $cm^{-1}$).

Beispiel 7 : Allyl-bicyclo[2.2.1]hept-5-en-2,3-dicarbonsäure-N-benzylimid.

102 g Allyl-bicyclo[2.2.1]hept-5-en-2,3-dicarbonsäureanhydrid und 85,6 g Benzylamin werden 2 Stunden lang unter Rückfluss gekocht. Danach destilliert man Wasser und überschüssiges Benzylamin ab (39 ml bei 105 °C und 4 132 Pa).

Die Destillation bei Kp = 170-184 °C und 10,7 Pa ergibt 117,35 g (80 % der Theorie) eines gelben Oels mit $n_D^{20}$ = 1,5 612 ; $\eta_{25}$ = 17,3 Pa.s.

Analyse

| | | | |
|---|---|---|---|
| berechnet für $C_{19}H_{19}NO_2$ : | % C 77,79 | % H 6,53 | % N 4,87 |
| gefunden : | % C 77,5 | % H 6,7 | % N 5,1. |

Beispiel 8 : N,N'-Aethylen-bis-(allyl-bicyclo[2.2.1]hept-5-en-2,3-dicarbonsäureimid).

Man legt 204 g Allyl-bicyclo[2.2.1]hept-5-en-2,3-dicarbonsäureanhydrid vor und tropft unter Rühren 30 g Aethylendiamin ein. Die Temperatur steigt bis 130 °C an. Man steigert die Temperatur auf 180 °C ; hierbei destillieren 14 ml Wasser ab. Danach erhitzt man weitere 2 Stunden auf 200 °C unter einem Druck von 9,3 Pa. Man erhält 210 g eines gelben, bei Raumtemperatur festen Harzes mit einem Erweichungspunkt von 56 °C, gemessen auf der Kofler-Heizbank. Molekulargewicht (Zahlenmittel)

$\bar{M}_n = 504$, (Gewichtsmittel) $\bar{M}_w = 1\,204$, bestimmt nach der Methode der Gelpermeationschromatographie.

Analyse
berechnet für $C_{26}H_{28}N_2O_4$ : % C 72,20 % H 6,53 % N 6,48
gefunden : % C 71,7 % H 6,5 % N 6,4.

Nach der Polymerisation während 12 Stunden bei 240 °C erhält man einen Festkörper mit einer Glasumwandlungstemperatur von 354 °C.

Beispiel 9 : N,N'-Hexamethylen-bis-(allyl-bicyclo[2.2.1]hept-5-en-2,3-dicarbonsäureimid).

Eine Mischung aus 204 g Allyl-bicyclo [2.2.1] hept-5-en-2,3-dicarbonsäureanhydrid und 58 g Hexamethylendiamin wird im Verlaufe von 3 Stunden unter Rühren am absteigenden Kühler auf 175 °C erhitzt. Danach wird der Druck auf 1 866 Pa reduziert und die Mischung eine weitere Stunde bei 175 °C gerührt. Man erhält 235 g eines bernsteinfarbenen Harzes, das bei Raumtemperatur gerade noch flüssig ist.

Analyse
berechnet für $C_{30}H_{36}N_2O_4$ : % C 73,74 % H 7,43 % N 5,73 $\bar{M}_n = 560$,
gefunden : % C 73,4 % H 7,4 % N 5,5. $\bar{M}_w = 1\,173$.

Beispiel 10 : N,N'-Dodecamethylen-bis-(allyl-bicyclo[2.2.1]hept-5-en-2,3-dicarbonsäureimid).

100,66 g Allylnadicanhydrid und 49,34 g 1,12-Diaminododecan werden stufenweise auf 200 °C, zuletzt unter Vakuum, erhitzt. Man erhält 140 g eines zähflüssigen, bernsteinfarbenen Harzes.

Analyse
berechnet für $C_{36}H_{50}O_4N_2$ : % C 75,22 % H 8,77 % N 4,87
gefunden : % C 75,8 % H 8,75 % N 5,1.

Beispiel 11 : Bis-[4-(allyl-bicyclo[2.2.1]hept-5-en-2,3-dicarbonsäure-imidophenyl)-methan].

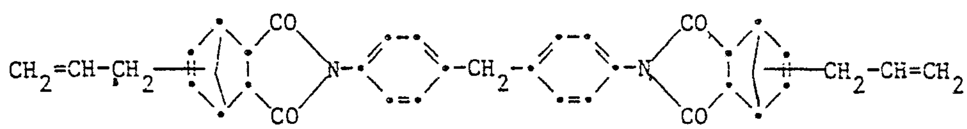

204 g Allyl-bicyclo[2.2.1]hept-5-en-2,3-dicarbonsäuranhydrid und 99 g 4,4'-Diaminodiphenylmethan werden im Vakuum auf 200 °C erhitzt und 1 Stunde gehalten. Man erhält 280 g eines braunen Festharzes mit einem Erweichungspunkt von 104 °C, einer Viskosität von 0,425 Pa.s bei 200 °C und einer Säurezahl von 0.

Analyse
berechnet für $C_{37}H_{34}N_2O_4$ : % C 77,87 % H 6,01 % N 4,91 $\bar{M}_n = 977$,
gefunden : % C 78,2 % H 6,1 % N 5,0 $\bar{M}_w = 4\,718$.

Beispiel 12

218 g Methallyl-bicyclo[2.2.1]hept-5-en-2,3-dicarbonsäureanhydrid (Kp = 139-142 °C bei 20 Pa) und 99 g 4,4'-Diaminodiphenyl-methan werden im Vakuum auf 200 °C erhitzt und 1 Stunde bei dieser Temperatur gehalten. Man erhält 295 g eines braunen Festharzes mit einem Erweichungspunkt von 98 °C und einer Säurezahl von 2 mg KOH/g.

Analyse
berechnet für $C_{38}H_{36}N_2O_4$ : % C 78,04 % H 6,12 % N 4,79
gefunden : % C 78,3 % H 6,1 % N 4,8.

Beispiel 13

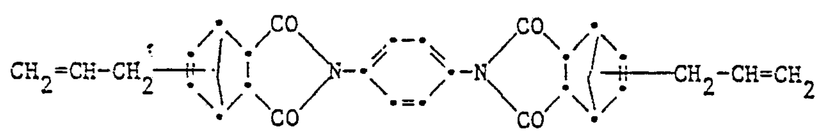

Man erhitzt 204 g Allyl-bicyclo[2.2.1]hept-5-en-2,3-dicarbonsäureanhydrid und 54,0 g 1,4-Phenylendiamin bei 2 000 Pa unter Abdestillieren des Reaktionswassers auf 220 °C. Man erhält 228 g (95 % d. Th.) eines dunkelbraunen Festharzes mit einem Erweichungspunkt von 140 °C.

Analyse
berechnet für $C_{30}H_{28}N_2O_4$ : % C 74,98 % H 5,87 % N 5,83
gefunden : % C 74,7 % H 6,1 % N 6,1.

Beispiel 14

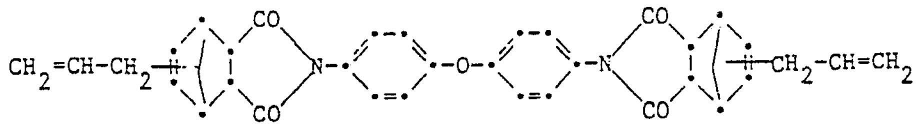

408 g Allyl-bicyclo[2.2.1]hept-5-en-2,3-dicarbonsäureanhydrid und 204,3 g 4,4'-Diaminodiphenyläther (Fp. = 188-190 °C, Zers., 98 %ig) werden auf 200 °C erhitzt. Dabei destillieren 35 $cm^3$ Wasser ab. Man erniedrigt den Druck auf 40 Pa, erhitzt auf 220 °C und hält diese Temperatur eine Stunde. Man erhält 555,8 g eines dunkelbraunen Festharzes (96,5 % d. Th.) mit einem Erweichungspunkt von 142 °C.
Analyse
berechnet für $C_{36}H_{32}N_2O_5$ : % C 75,51 % H 5,63 % N 4,89 %
gefunden : % C 75,5 % H 5,7 % N 4,9.

Beispiel 15

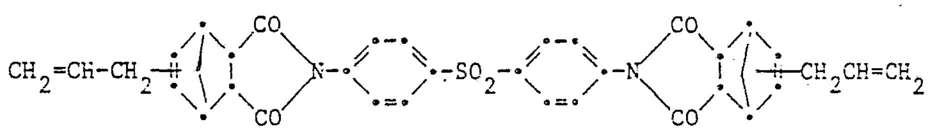

Man erhitzt 102 g Allyl-bicyclo[2.2.1]hept-5-en-2,3-dicarbonsäureanhydrid und 62 g 4,4'-Diaminodiphenylsulfon (Fp. = 174-176 °C) auf 180 °C, erniedrigt den Druck auf 27 Pa und hält 1 Stunde unter diesen Bedingungen. Man erhält 135,6 g eines braunen festen Harzes (87,5 % d. Th.) mit einem Erweichungspunkt von 108 °C.

Herstellung von vernetzten Polymeren

Die erfindungsgemässen Verbindungen haben zwei oder mehr olefinische Doppelbindungen im Molekül, die sie zur Polymerisation befähigen. Beim Erhitzen erhält man vernetzte Polymere mit wertvollen physikalischen Eigenschaften.

Anwendungsbeispiele

Beispiel I

Das gemäss Beispiel 8 hergestellte Harz giesst man als dünnflüssige Schmelze in eine auf 200 °C vorgewärmte Stahlform von 12 × 12 × 0,4 $cm^3$ und härtet 12 Stunden bei 200 °C und 12 Stunden bei 240 °C aus. Nach dem Abkühlen zerschneidet man die Platte zu Prüfstäben. Folgende Eigenschaften werden daran bestimmt :

| | |
|---|---|
| Biegefestigkeit nach DIN 53 452 : | 95,4 $N/mm^2$ |
| Schlagbiegefestigkeit nach DIN 53 455 : | 7,54 $kJ/m^2$ |
| Wasseraufnahme 1 Stunde bei 100 °C : | 0,25 Gew.% |
| Vicat-Erweichungstemperatur nach DIN 53 460 : | > 250 °C |
| Gewichtsverlust nach 30 Tagen bei 250 °C an der Luft : | 2,23 Gew.%. |

Metallverklebung : Blechstreifen (170 × 25 $mm^2$) aus Anticorodal B werden mit der Harzschmelze aus Beispiel 8 in einer Ueberlappung von 12,5 mm verklebt und wie oben beschrieben ausgehärtet. Die Zugscherfestigkeit nach DIN 53 283 beträgt 5,7 $N/mm^2$.

Beispiel II

Das gemäss Beispiel 9 hergestellte Imid giesst man als heisses, dünnflüssiges Harz in eine Stahlform von 12 × 12 × 0,4 $cm^3$ und härtet 1 Stunde bei 200 °C, 1 Stunde bei 220 °C und 12 Stunden bei 240 °C aus. Nach dem Abkühlen schneidet man Prüfstäbe aus der Platte. Folgende Eigenschaften werden daran gemessen :

| | |
|---|---|
| Biegefestigkeit nach DIN 53 452 : | 89,8 $N/mm^2$ |
| Durchbiegung : | 4,3 mm |
| Schlagbiegefestigkeit nach DIN 53 455 : | 9,2 $kJ/m^2$ |
| Vicat-Erweichungstemperatur nach DIN 53 460 : | 247 °C |
| Wasseraufnahme (1 Stunde bei 100 °C) : | 0,42 Gew.% |
| Zugscherfestigkeit auf Anticorodal gemäss DIN 53 283 : | 7,2 $N/mm^2$. |

Beispiel III :

Das gemäss Beispiel 10 hergestellte Imid wird in eine Stahlform von 12 × 12 × 0,4 $cm^3$ gegossen und während 15 Stunden bei 220 °C und während 4 Stunden bei 240 °C gehärtet. Prüfstäbe aus der erhaltenen Platte zeigen folgende Eigenschaften :

| | |
|---|---|
| Biegefestigkeit : | 89,7 $N/mm^2$ |
| Durchbiegung : | 6,1 mm |
| Schlagzähigkeit : | 14,4 $kJ/m^2$ |
| Erweichungstemperatur nach Vicat | 152 °C |
| Wasseraufnahme (1 Stunde bei 100 °C) : | 0,43 Gew.% |

Beim Verkleben von Aluminiumblech unter den gleichen Härtungsbedingungen, wobei die beiden zu verklebenden Bleche sich mit 25 × 12 $mm^2$ überlappen, erhält man eine ausgezeichnete Zugscherfestigkeit von 10 ± 0,5 $N/mm^2$ (nach DIN 53 283).

Beispiel IV :

Das gemäss Beispiel 11 hergestellte Giessharz wird in eine Stahlform von 12 × 12 × 0,4 $cm^3$ gegossen und während 6 Stunden bei 220 °C und während 14 Stunden bei 240 °C gehärtet. Prüfstäbe zeigen die folgenden Eigenschaften :

| | |
|---|---|
| Biegefestigkeit : | 103,1 $N/mm^2$ |
| Durchbiegung : | 2,3 mm |
| Schlagzähigkeit : | 12,5 $kJ/m^2$ |
| Erweichungstemperatur nach Vicat : | > 260 °C |
| Wasseraufnahme (1 Stunde bei 100 °C) : | 0,27 Gew.% |
| Zugscherfestigkeit auf Anticorodal gemäss DIN 53 283 : | 5,3 $N/mm^2$. |

Hitzebeständigkeit : thermische Analyse an der Luft bei einer Aufheizgeschwindigkeit von 2 °C/Min. : Beginn der Zersetzung bei 390 °C, 10 % Gewichtsverlust bei 420 °C.

| | |
|---|---|
| Gewichtsverlust nach 30 Tagen bei 250 °C : | 2,4 Gew.% |
| Gewichtsverlust nach 30 Tagen bei 275 °C : | 3,8 Gew.%. |
| Biegefestigkeit nach 30 Tagen bei 250 °C : | 104,9 $N/mm^2$ |
| Biegefestigkeit nach 30 Tagen bei 275 °C : | 68,5 $N/mm^2$. |

| | |
|---|---|
| Dielektrische Eigenschaften : | |
| Dielektrizitätskonstante ε bei 22 °C : | 3,3 |
| Dielektrizitätskonstante ε bei 250 °C : | 2,9 |
| dielektrischer Verlustfaktor nach DIN 53 483 bei 22 °C : | 0,16 % |
| dielektrischer Verlustfaktor nach DIN 53 483 bei 250 °C : | 0,42 % |
| spezifischer Durchgangswiderstand nach DIN 53 482 bei 22 °C : | 2,8 × $10^{16}$ ohm.cm |
| bei 250 °C : | 1,3 × $10^{13}$ ohm.cm. |

Beispiel V :

Das gemäss Beispiel 14 hergestellte Imidharz wird vergossen und gehärtet, wie dies im vorhergehenden Beispiel beschrieben ist. Prüfstäbe zeigen die folgenden Eigenschaften :

| | |
|---|---|
| Biegefestigkeit : | 99,3 $N/mm^2$ |
| Durchbiegung : | 3,1 mm |
| Schlagbiegefestigkeit : | 12,9 $kJ/m^2$ |
| Erweichungstemperatur nach Vicat : | > 260 °C |
| Gewichtsverlust nach 30 Tagen bei 275 °C : | 3,89 Gew.% |
| Wasseraufnahme (1 Stunde bei 100 °C) : | 0,55 Gew.% |
| Zugscherfestigkeit auf Anticorodal gemäss DIN 53 283 : | 6,2 $N/mm^2$. |

**Patentansprüche**

1. Imide der Formel I

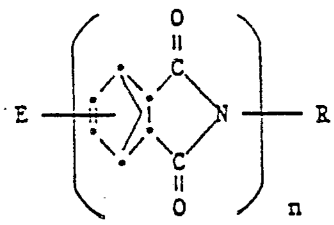

(I)

worin E Allyl oder Methallyl und n 1 oder 2 bedeuten und R, falls n 1 bedeutet, für Wasserstoff, Alkyl mit 1-12 C-Atomen, Alkenyl mit 3-6 C-Atomen, Cycloalkyl mit 5-8 C-Atomen, Aryl mit 6-10 C-Atomen oder Benzyl oder, falls n 2 bedeutet, für $-C_mH_{2m}-$ mit m = 2-20, Arylen mit 6-10 C-Atomen oder für eine Gruppe der Formel II

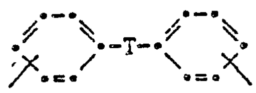

(II)

worin T Methylen, Isopropyliden, CO, O, S oder $SO_2$ bedeutet, steht.

2. Imide der Formel I gemäss Anspruch 1, worin E die Allylgruppe bedeutet.

3. Imide der Formel I gemäss Anspruch 1, worin E die Allylgruppe bedeutet und R, falls n = 1 ist, für Wasserstoff, Alkyl mit 1-8 C-Atomen, Allyl, Cyclohexyl, Phenyl oder Benzyl steht, oder, falls n = 2, für $-(CH_2)_m-$ mit m = 2-12, m-oder p-Phenylen oder für eine Gruppe der Formel II

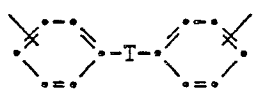

(II)

steht, worin T die Methylengruppe, O oder $SO_2$ bedeutet.

4. Imide der Formel I gemäss Anspruch 1, worin E die Allylgruppe, n die Zahl 2 und R $-(CH_2)_2-$, $-(CH_2)_6-$

oder 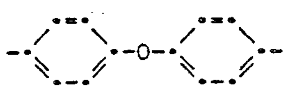 und insbesondere 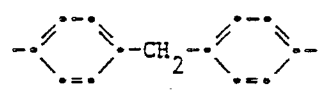 .

bedeuten.

5. Verfahren zur Herstellung von Imiden der Formel I nach Anspruch 1, dadurch gekennzeichnet, dass man ein Anhydrid der Formel III

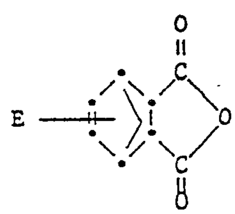

(III)

bei erhöhter Temperatur und unter Abdestillieren des bei der Reaktion entstehenden Wassers mit einer Verbindung der Formel IV

$$(H_2N)_n-R \qquad (IV)$$

umsetzt, wobei E, R und n die im Anspruch 1 angegebene Bedeutung haben.

6. Polymere, die dadurch erhältlich sind, dass man ein Imid der Formel I genäss Anspruch 1 während 6 bis 24 Stunden auf eine Temperatur zwischen 180 und 300 °C erhitzt.

7. Polymere nach Anspruch 6, dadurch gekennzeichnet, dass man eine Verbindung der Formel I, worin E die Allylgruppe, n die Zahl 2 und R

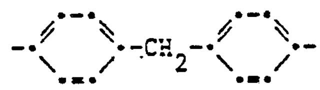

bedeuten, während 6 bis 12 Stunden auf 240 bis 250 °C erhitzt.

**Claims**

1. An imide of the formula I

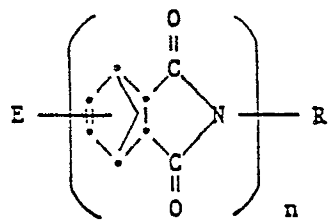

(I)

in which E is allyl or methallyl, n is 1 or 2 and, if n is 1, R is hydrogen, alkyl having 1-12 C atoms, alkenyl having 3-6 C atoms, cycloalkyl having 5-8 C atoms, aryl having 6-10 C atoms or benzyl or, if n is 2, R is $-C_mH_{2m}-$, in which m is 2-20, arylene having 6-10 C atoms or a group of the formula II

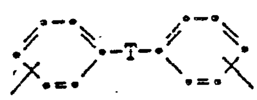

(II)

in which T is methylene, isopropylidene, CO, O, S or $SO_2$.

2. An imide of the formula I according to claim 1, in which E is the allyl group.

3. An imide of the formula I according to claim 1, in which E is the allyl group and, if n is 1, R is hydrogen, alkyl having 1-8 C atoms, allyl, cyclohexyl, phenyl or benzyl, or, if n is 2, R is $-(CH_2)_m-$, in which m is 2-12, m- or p-phenylene or a group of the formula II

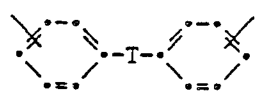

(II)

in which T is the methylene group, O or $SO_2$.

4. An imide of the formula I according to claim 1, in which E is the allyl group, n is 2 and R is $-(CH_2)_2-$, $-(CH_2)_6-$

or 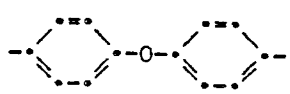 or in particular 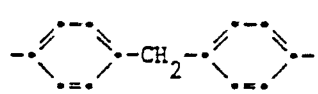 .

5. A process for the preparation of an imide of the formula I, which process comprises reacting an anhydride of the formula III

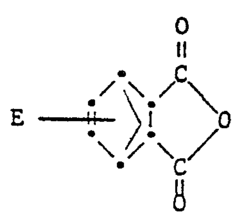

(III)

with a compound of the formula IV

$$(H_2N)_n-R \qquad (IV)$$

in which E, R and n are as defined for formula I, at elevated temperature, the water formed during the reaction being distilled off.

6. A polymer which is obtainable by heating an imide of formula I according to claim 1 to a temperature in the range from 180 to 300 °C for 6 to 24 hours.

7. A polymer according to claim 6, which is obtainable by heating a compound of formula I in which E is the allyl group, n is 2 and R is

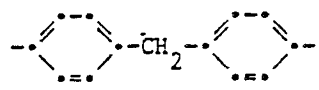

to 240 to 250 °C for 6 to 12 hours.

**Revendications**

1. Imides qui répondent à la formule (I) :

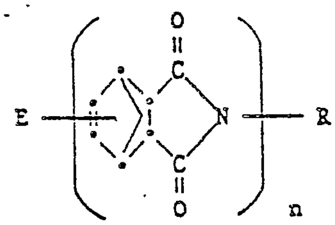

(I)

dans laquelle E représente un groupe allyle ou méthallyle, n vaut 1 ou 2, et R, lorsque n est égal à 1, représente un atome d'hydrogène, un groupe alkyle en $C_1$-$C_{12}$, alcényle en $C_3$-$C_6$, cycloalkyle en $C_5$-$C_8$, aryle en $C_6$-$C_{10}$ ou benzyle ou, lorsque n est égal à 2, représente un groupe $—C_mH_{2m}—$ où m est un nombre de 2 à 20, ou un groupe arylène en $C_6$-$C_{10}$, ou un groupe de formule (II) :

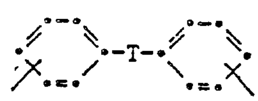

(II)

dans laquelle T représente un groupe méthylène, isopropylidène, —CO—, —O—, —S— ou $—SO_2—$.

2. Imides selon la revendication 1 caractérisés en ce que E représente le groupe allyle.

3. Imides selon la revendication 1, caractérisés en ce que E représente le groupe allyle et R, lorsque n est égal à 1, représente un atome d'hydrogène, un groupe alkyle en $C_1$-$C_8$, allyle, cyclohexyle, phényle ou benzyle ou, lorsque n est égal à 2, représente un groupe $—(CH_2)_m—$ où m est un nombre de 2 à 12, un groupe m- ou p- phénylène ou un groupe de formule II :

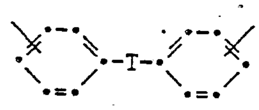

(II)

dans laquelle T représente le groupe méthylène, —O— ou $—SO_2—$.

4. Imides selon la revendication 1, caractérisés en ce que E représente le groupe allyle, n vaut 2 et R représente le groupe $—(CH_2)_2—$, $—(CH_2)_6—$ ou

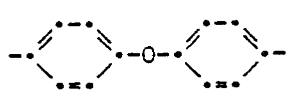 et en particulier le groupe 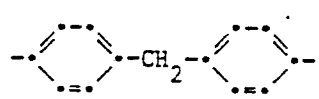 .

5. Procédé de préparation d'imides de formule I selon l'une quelconque des revendications 1 à 4, caractérisé en ce que l'on fait réagir un anhydride de formule III :

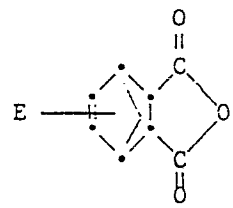

(III)

à haute température et en éliminant par distillation l'eau formée par la réaction, sur un composé de formule IV :

$$(H_2N)_n\text{—}R \qquad (IV)$$

formules dans lesquelles E, R et n sont tels que définis dans la revendication 1.

6. Polymères caractérisés en ce qu'ils sont obtenus par chauffage à une température de 180 à 300 °C, durant 6 à 24 h, d'un imide de formule I selon l'une quelconque des revendications 1 à 4.

7. Polymères selon la revendication 6, caractérisés en ce qu'ils sont obtenus par chauffage à 240-250 °C, durant 6 à 12 h, d'un imide de formule I dans laquelle E représente un groupe allyle, n vaut 2 et R représente le groupe :